(19) Europäisches Patentamt
European Patent Office
Office européen des brevets

(11) Publication number: **0 257 151 B1**

# (12) EUROPEAN PATENT SPECIFICATION

(45) Date of publication of patent specification: **29.01.92** (51) Int. Cl.[5]: **C07D 249/20**

(21) Application number: **86306514.0**

(22) Date of filing: **22.08.86**

The file contains technical information submitted after the application was filed and not included in this specification

## (54) Method for preparing 2-phenylbenzotriazoles and 2-phenylbenzotriazole-N-oxides.

(43) Date of publication of application:
**02.03.88 Bulletin 88/09**

(45) Publication of the grant of the patent:
**29.01.92 Bulletin 92/05**

(84) Designated Contracting States:
**CH DE FR GB IT LI**

(56) References cited:
**EP-A- 0 130 938**

**CHEMICAL ABSTRACTS, vol. 98, no. 11, 14th March 1983, page 549, abstract no. 89257h, Columbus, Ohio, US; J. ROSEVEAR et al.: "The reduction of some o-nitrophenylazo dyes with glucose: a general synthesis of 2-aryl-2H-benzotriazole 1-oxides"**

**"Methoden der Organischen Chemie", (HOUBEN-WEYL), 1981, edition 4, vol. IV/1d, Georg Thieme Verlag, Stuttgart, DE**

(73) Proprietor: **CHEMIPRO KASEI KAISHA, LTD.**
**No. 2-15, Gokodori 5-chome Chuo-ku**
**Kobe-shi Hyogo-ken(JP)**

(72) Inventor: **Seino, Shuichi**
**No. 3-6, Karibadai 5-chome Nishi-ku**
**Kobe-shi Hyogo-ken(JP)**

(74) Representative: **Perry, Robert Edward et al**
**GILL JENNINGS & EVERY 53-64 Chancery Lane**
**London WC2A 1HN(GB)**

EP 0 257 151 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method for preparing 2-phenylbenzotriazoles and 2-phenylbenzotriazole-N-oxides.

2-Phenylbenzotriazoles of formula I, wherein $R_1$ is H, Cl, $C_{1-4}$ alkyl, $C_{1-4}$ alkoxy, COOH or $SO_3H$; $R_2$ is H, Cl, $C_{1-4}$ alkyl or $C_{1-4}$ alkoxy; $R_3$ is H, Cl, $C_{1-12}$ alkyl, $C_{1-4}$ alkoxy. phenyl, ($C_{1-8}$ alkyl)phenyl, phenoxy or phenyl($C_{1-8}$ alkyl); $R_4$ is H, Cl, OH or $C_{1-4}$ alkoxy; and $R_5$ is H, $C_{1-12}$ alkyl or phenyl($C_{1\text{-}4}$ alkyl), are known, and have utility as ultra-violet absorbers which can be added to, say, plastics, paints and oils. 2-Phenylbenzotriazole-N-oxides of formula II, wherein $R_1$, $R_2$, $R_3$, $R_4$ and $R_5$ are as defined above, are valuable intermediates in the preparation of the 2-phenylbenzotriazoles (I).

These known compounds have previously been produced by chemically or electrolytically reducing nitroazobenzenes of formula III, wherein $R_1$, $R_2$, $R_3$, $R_4$ and $R_5$ are as defined above. However, the known methods are not always satisfactory.

JP-A-37/5934 and US-A-3773751 disclose chemically reducing nitroazobenzenes (III) in an alcoholic sodium hydroxide solution with zinc powder. The yield is satisfactory but zinc sludge is produced, causing waste water contamination.

US-A-2362988 discloses chemical reduction of nitroazobenzenes (III) using ammonium sulphide, an alkali sulphide, zinc-ammonia, hydrogen sulphide-sodium or zinc-hydrochloric acid. This method produces a large amount of sulphite or zinc salts, thus contaminating waste water. Further, sulphites generates sulphurous acid gas, and the sulphide reducing agents generate poisonous hydrogen sulphide.

JP-A-51138679 and JP-A-51138680 disclose reduction using hydrogen under pressure. JP-A-5088072 discloses reduction using hydrazine. These methods give poor yields and are not economic. Further, it is impossible to obtain the desired product in high purity, owing to side-reactions. For example, when producing a chlorine-containing product, dechlorination occurs.

Chem. Abs. vol. 98, no. 15, 11/04/83, page 643, abstract no. 126102b and JP-A-57 167 976 disclose a method for reducing (III) → (I)/(II) using formaldehyde as a reducing agent.

According to a first aspect of the present invention, a method for preparing a 2-phenylbenzotriazole (I) as defined above comprises reducing a nitroazobenzene (III) as defined above with a saccharide in the presence of a hydrogen transfer catalyst and base.

According to a second aspect of the invention, a method for preparing a 2-phenylbenzotriazole (I) as defined above comprises reducing 2-phenylbenzotriazole-N-oxide (II) as defined above with, per mol thereof, 0.5 to 1.5 mol saccharide in the presence of a hydrogen transfer catalyst and base.

According to a third aspect of the invention, a method for preparing a 2-phenylbenzotriazole-N-oxide (II) as defined above comprises reducing nitroazobenzene (III) as defined above with, per mol thereof, 0.5 to 0.8 mol saccharide in the presence of a hydrogen transfer catalyst and base.

The processes of the invention give technically and economically satisfactory results, without causing environmental pollution.

The method according to the first aspect of the invention, i.e. III→I, can be carried out in either one or two steps, depending on the conditions such as temperature and the amount of the saccharide which is used. For a one-step reaction, a suitable temperature is 60 to 80 C, 1 to 2 mols of the saccharide reducing agent being used per mol of the starting material (III).

The two-step method is sometimes advantageous in terms of product purity and yield, despite the use of two steps. In the first step, 0.5 to 0.8 mol saccharide is preferably used per mol starting material (III); the temperature is suitably 20 to 80 C. In the second step, 0.5 to 1.5 mol saccharide are preferably used per mol of the resulting intermediate (II); a suitable temperature is 60 to 100 C. These preferred temperatures are also preferred for the independent III→I and II→I methods according to the second and third aspects of the invention.

In order that the III→I, III→II or II→I process should proceed smoothly, it is preferably conducted in aqueous solution, or in an inert solvent such as an alkali, toluene, acetone, dimethyl sulphoxide or acetonitrile; a mixture of the inert solvent with water may also be used. If necessary or desired, a surface-active agent or a phase-transfer catalyst may also be used.

The following are examples of nitroazobenzenes of formula III:
2-nitro-4-chloro-2′-hydroxy-3′-t-butyl-5′-methyl-azobenzene,
2-nitro-2′-hydroxy-5′-methylazobenzene,
2-nitro-2′-hydroxy-5′-t-octylazobenzene,
2-nitro-2′-hydroxy-5′-t-butylazobenzene,
2-nitro-4-chloro-2′-hydroxy-3′, 5′-di-t-butylazobenzene,
2-nitro-2′-hydroxy-3′, 5′-di-t-amylazobenzene,

2-nitro-2′-hydroxy-3′, 5′-di-t-butylazobenzene,
2-nitro-2′-hydroxy-3′-t-butyl-5′-methylazobenzene,
2-nitro-2′, 4′-dihydroxyazobenzene,
2-nitro-4-chloro-2′, 4′-dihydroxyazobenzene,
2-nitro-2′-hydroxy-4′-methoxyazobenzene,
2-nitro-4-chloro-2′-hydroxy-3′, 5′-di-t-amylazobenzene,
2-nitro-2′-hydroxy-5′-t-amylazobenzene,
2-nitro-4-chloro-2′-hydroxy-5′-t-amylazobenzene,
2-nitro-2′-hydroxy-3′, 5′-di(α,α-dimethylbenzyl) azobenzene,
2-nitro-4-chloro-2′-hydroxy-3′, 5′-di (α, α-dimethylbenzyl)-azobenzene,
2-nitro-2′-hydroxy-3′-α-methylbenzyl-5′-methylazobenzene,
2-nitro-4-chloro-2′-hydroxy-3′-α-methylbenzyl-5′-methylazobenzene,
2-nitro-2′-hydroxy-5′-n-dodecylazobenzene,
2-nitro-4-chloro-2′-hydroxy-5′-n-dodecylazobenzene,
2-nitro-2′-hydroxy-3′, 5′-di-t-octylazobenzene,
2-nitro-4-chloro-2′-hydroxy-3′, 5′-di-t-octylazobenzene,
2-nitro-4-chloro-2′-hydroxy-5′-t-octylazobenzeze,
2-nitro-4-methyl-2′-hydroxy-5′-methylazobenzene.
2-nitro-4-methyl-2′-hydroxy-3′-t-butyl-5-methylazobenzene,
2-nitro-4-n-butyl-2′-hydroxy-3′, 5′-di-t-butylazobenzene,
2-nitro-4-n-butyl-2′-hydroxy-3′-sec-butyl-5′-t-butylazobenzene.
2-nitro-4-t-butyl-2′-hydroxy-3′-sec-butyl-5′-t-butylazobenzene,
2-nitro-4,6-dichloro-2′-hydroxy-5′-t-butylazobenzene,
2-nitro-4,6-dichloro-2′-hydroxy-3′,5′-di-t-butylazobenzene, and
2-nitro-4-carboxy-2′-hydroxy-5-methylazobenene.

The following are examples of 2-phenylbenzotriazole-N-oxides of formula II:
2-(2-hydroxy-3-t-butyl-5-methylphenyl)-5-chlorobenzotriazole-N-oxide,
2-(2-hydroxy-3,5-di-t-butylphenyl)-5-chlorobenzotriazole-N-oxide,
2-(2-hydroxy-3,5-di-t-amylphenyl)benzotriazole-N-oxide,
2-(2-hydroxy-5-methylphenyl)benzotriazole-N-oxide,
2-(2-hydroxy-5-t-butylphenyl)benzotriazole-N-oxide,
2-(2-hydroxy-5-t-octylphenyl)benzotriazole-N-oxide,
2-(2-hydroxy-3,5-di-t-butylphenyl)benzotriazole-N-oxide,
2-(2-hydroxy-3-t-butylphenyl)benzotriazole-N-oxide,
2-(2,4-dihydroxyphenyl)benzotriazole-N-oxide,
2-(2,4-dihydroxyphenyl)-5-chlorobenzotriazole-N-oxide,
2-(2-hydroxy-4-methoxyphenyl)benzotriazole-N-oxide,
2-[2-hydroxy-3,5-di(α,α-dimethylbenzyl)phenyl]benzotriazole-N-oxide, and
2-(2-hydroxy-3-α-methylbenzyl-5-methylphenyl)benzotriazole-N-oxide.

The 2-phenylbenzotriazole-N-oxides (II) are preferably prepared by reduction of the nitroazobenzenes (III). Alternatively, for use in the II→I conversion according to the invention, they may be obtained by any other suitable process.

The saccharide reducing agent is, for example, glucose, fructose, sucrose, lactose or maltose. Glucose is most preferred.

A hydrogen transfer catalyst is a material which acts catalytically by receiving hydrogen from a reducing agent and giving hydrogen to a material to be reduced, or by giving hydrogen to a material to be reduced and then taking hydrogen from a reducing agent.

Examples of hydrogen transfer catalysts which can be used in this invention include 2,3-dichloro-1,4-naphthoquinone, 1,4-naphthoquinone, 1,2-naphthoquinone, 2,6-naphthoquinone, naphthoquinone having an alkyl or alkoxy substituent in the nucleus, 1,4-benzoquinone, 2-chloro-1,4-benzoquinone, 2,3-dichloro-5,6-dicyanobenzoquinone, chloroanil, tetrachloro-1,2-benzoquinone, 4,4′-diphenoquinone, 3,3′,5,5′-tetrachloro-4,4′-diphenoquinone, phenanthrenequinone, anthraquinone, anthraquinone substituted by an alkyl, alkoxy, amino, carboxyl or sulphonic acid group or a halogen atom, benzophenone, benzophenone substituted by an alkyl, alkoxy or hydroxyl group or a halogen atom, benzathrone, anthrone, 9-fluorenone, 9-xanthenone and various hydroquinones respectively corresponding to the above-mentioned quinones (for example, 2,3-dichloro-1,4-dioxynaphthalene corresponding to 2,3-dichloro-1,4-naphthoquinone). Preferred examples are 2,3-dichloro-1,4-naphthoquinone, benzoquinone, anthraquinone, 9-fluorenone, benzanthrone, hydroquinone and 1,4-hydroxynaphthalene. One or a mixture of two or more of such hydrogen transfer catalysts may be

used.

The hydrogen transfer catalyst is used generally in an amount of 0.2 to 30%, preferably 2 to 20%, by weight based on the weight of the starting material, i.e. nitroazobenzene (III) or 2-phenylbenzotriazole-N-oxide (II).

The base used in the present invention is, for example, sodium hydroxide or potassium hydroxide. Such a base is preferably used in an amount of 1 to 12 mol, more preferably 2 to 8 mol, per mol of the starting material, i.e. nitroazobenzene (III) or 2-phenylbenzotriazole-N-oxide (II).

The present invention is further illustrated by the following Examples.

Example 1

2-nitro-2′-hydroxy-3′, 5′-di-t-amylazobenzene (12.8 g) was added to a mixture of methanol (41 g), water (10 g) and 97% sodium hydroxide (11 g), and the resultant mixture was stirred at 65 °C for 30 minutes. Thereafter, 9-fluorenone (2.0 g) and then glucose (12 g) were added to the mixture at 60 to 65 °C over 4 hours. The resultant mixture was further stirred for 4 hours at the boiling point (70 °C), thus completing the reduction reaction.

Thereafter, water (50 ml) was added to the reaction liquor, and the reaction liquor was neutralized with 62 % sulfuric acid (13 g). The liquor thus neutralized was cooled to 20 °C to precipitate a crystal. The crystal thus obtained was separated by filtration, and the separated crystal was fully washed with water and further with methanol. The washed crystal was then dried, thus producing 9.9 g of 2-(2′-hydroxy-3′, 5′-di-t-amylphenyl)benzotriazole having a melting point of 77 to 79 °C at the yield of 85.0%.

Example 2

97% sodium hydroxide (9.6 g) was added to methanol (100 ml), and was stirred at 65 °C for 30 minutes. After cooling to 50 °C, 2-nitro-4-chloro-2′-hydroxy-3′-t-butyl-5′-methylazobenzene (11.6 g) was added to the resultant mixture over 30 minutes, and thereafter 2,3-dichloro-1,4-naphthoquinone (0.7 g) was added. Glucose (8 g) was added to the resultant mixture at 40 to 45 °C over one hour, and the mixture was stirred for one hour at 40 to 45 °C. As a result, almost all of the azobenzenes disappeared to produce 2-(2′-hydroxy-3′-t-butyl-5′-methylphenyl)-5-chlorobenzotriazole-N-oxide.

The system was heated, and was stirred at 64 to 67 °C for two hours. As a result, the N-oxide disappeared.

Thereafter, water (50 ml) was added to the reaction liquor, and the resultant reaction liquor was neutralized with 62% sulfuric acid (11 g) to precipitate a crystal. The precipitated crystal was separated by filtration, and the separated crystal was fully washed with hot water of 60 to 70 °C and further with a small amount of methanol. The washed crystal was then dried, thus obtaining 9.4 g of 2-(2′-hydroxy-3′-t-butyl-5′-methylphenyl)-5-chlorobenzotriazole having a melting point of 138 to 140 °C at the yield of 89.5%.

Example 3

The same procedure as in Example 2 was repeated, except that 2-nitro-4-chloro-2′-hydroxy-3′-t-butyl-5′-methylazobenzene (11.6 g) was replaced respectively by (a) 2-nitro-4-chloro-2′-hydroxy-3′, 5′-di-t-butylazobenzene (13.0 g) and (b) 2-nitro-2′-hydroxy-3′, 5′-di-t-amylazobenzene (12.8 g).

The products thus obtained and their properties are as follows:

(a) 2-(2′-hydroxy-3′, 5′-di-t-butylphenyl)-5-chlorobenzotriazole:
Yield: 10.5 g (88.0%), Melting Point: 154 to 155.5 °C
(b) 2-(2′-hydroxy-3′, 5′-di-t-amylphenyl)benzotriazole:
Yield: 10.1 g (86.0 %), Melting Point: 77 to 79 °C.

Example 4

97 % sodium hydroxide (8.2 g) was added and dissolved in a mixture of methanol (60 ml) and water (20 ml). 2-nitro-4-chloro-2′-hydroxy-3′-t-butyl-5′-methylazobenzene (11.6 g) was then added to the resultant solution at 50 to 60 °C over 30 minutes while stirring, and thereafter 2,3-dichloro-1,4-naphthoquinone (0.3 g) and 9-fluorenone (0.4 g) were added to the solution. Glucose (8 g) was then added to the resultant mixture at 40 to 50 °C over two hours, and the mixture was stirred for one hour at the same temperature. As this result, almost all of the azobenzene disappeared to produce 2-(2-hydroxy-3-t-butyl-5-methylphenyl)-5-chlorobenzotriazole-N-oxide.

The system was heated, and was stirred at 66 to 68 °C (boiling point) for 5 hours. As a result, the N-oxide

disappeared.
Thereafter, water (50 ml) was added to the reaction liquor, and the resultant reaction liquor was neutralized with 62 % sulfuric acid (10 g) to precipitate a crystal. The precipitated crystal was separated by filtration, and the separated crystal was fully washed with hot water of 60 to 70 °C and further with a small amount of methanol. The washed crystal was then dried, thus obtaining 8.7 g of 2-(2′-hydroxy-3′-t-butyl-5′-methylphenyl)-5-chloronzotriazole having a melting point of 138 to 140 °C at the yield of 82.9 %.

Example 5

Water (70 ml), 97% sodium hydroxide (5.2 g), 2-nitro-2′ -hydroxy-5′-methylazobenzene (12.9 g) and toluene (10 ml) were mixed and heated to 60 °C. After stirring, hydroquinone (0.6 g) was added, and glucose (5.0 g) was added to the mixture over one hour at 40 to 45 °C. The mixture was further stirred for two hours, and the azobenzene disappeared. The reaction liquor was neutralized with 62 % sulfuric acid (5.8 g), and was cooled to 20 °C to precipitate a crystal. The crystal thus precipitated was separated by filtration to obtain a wet product 12 g of 2-(2′-hydroxy -5′-methylphenyl)benzotriazole-N-oxide (dry weight: 10.8 g, yield: 90.0%, and melting point: 138 to 140 °C).

To the wet product 12 g thus obtained, were added methanol (60 ml), water (30 ml), 97 % sodium hydroxide (13.0 g) and 9-fluorenone (0.5 g), and glucose (5.5 g) was further added to the mixture over one hour while stirring at 50 to 55 °C. The mixture was reacted while stirring at 75 °C (boiling point) for 5 hours. As a result, the N-oxide disappeared.
The reaction liquor was neutralized with 62 % sulfuric acid (19.8 g) to pH 8 to precipitate a crystal. The precipitated crystal was separated by filtration, and the separated crystal was fully washed with water and further with methanol. The washed crystal was then dried, thus obtaining 9.4 g of 2-(2′-hydroxy-5′-methylphenyl) benzotriazole having a melting point of 128 to 130 °C at the yield of 92.8 %.

Example 6

The same procedure as in Example 5 was repeated, except that 9-fluorenone (0.5 g) was replaced respectively by (a) benzanthrone (1.0 g), (b) benzophenone (1.5 g) and (c) anthrone (1.0 g) to produce 2-(2′-hydroxy-5′-methylphenyl)benzotriazole.

The products were obtained with the following yields:
(a) Yield: 9.0 g (88.8 %), Melting Point: 128~130 °C;
(b) Yield: 8.0 g (79.9 %), Melting Point: 127.7 ~130 °C;
and (c) Yield: 8.2 g (80.9 %), Melting Point: 128 ~130 °C.

Example 7

97 % sodium hydroxide (14.4 g) was added to a mixture of methanol (72 ml) and water (36 ml). 2-nitro-2′-hydroxy-5′ -t-butylazobenzene (15.0 g) was then added to the resultant mixture and the mixture was heated to 45~50 °C. Hydroquinone (0.4 g) and then glucose (5.0 g) were added to the heated mixture over 30 minutes while stirring. The mixture was further stirred for one hour. As a result, the azobenzene disappeared to produce 2-(2′-hydroxy-5′-t-butylphenyl)benzotriazole-N-oxide.

9-fluorenone (0.7 g) was then added to the reaction liquor thus obtained, and the liquor was heated to 55~60 °C. Thereafter, glucose (6.0 g) was added to the reaction liquor over 30 minutes, and the reaction was conducted at 75 °C (boiling point) for 6 hours. As a result, the N-oxide disappeared.

Thereafter, pH of the reaction liquor was made to 8 with 62 % sulfuric acid (19.0 g) to precipitate a crystal. The precipitated crystal was separated by filtration, and the separated crystal was fully washed with water and further with methanol. The washed crystal was then dried, thus obtaining 11.6 g of 2-(2′-hydroxy-5′-t-butylphenyl)benzotriazole.

Example 8

The same procedure as in Example 7 was repeated, except that 2-nitro-2′-hydroxy-5′-t-butylazobenzene (15.0 g) was replaced by 2-nitro-2′-hydroxy-5′-t-octylazobenzene (17.7 g), thus producing 13.7 g of 2-(2′-hydroxy-5′-t--octylphenyl)benzotriazole having a melting point of 103 to 105 °C at the yield of 85.0 %.

Example 9

A mixture of methanol (60 ml), water (30 ml), 97% sodium hydroxide (12.4 g) and 2-nitro-2′-hydroxy-5′-methyl azobenzene (12.9 g) was heated and stirred at 45~50° C. 9-fluorenone (1.0 g) and then glucose (5.5 g) were added to the resultant mixture over 30 minutes while stirring. The mixture was further stirred at 75° C (boiling point) for 7 hours. As a result, the azobenzene disappeared to produce 2-(2′-hydroxy-5′-methylphenyl)benzotriazole-N-oxide.

Glucose (6 g) was then added to the reaction liquor over 30 minutes and the reaction was conducted at 75° C (boiling point) for further 6 hours. As a result, the above-prepared N-oxide disappeared.

Thereafter, water 50 ml was added to the reaction liquor, and the resultant reaction liquor was neutralized with 62 % sulfuric acid (15 g) to precipitate a crystal. The precipitated crystal was separated by filtration, and the separated crystal was fully washed with water and further with methanol. The washed crystal was then dried, thus obtaining 9.6 g of 2-(2′-hydroxy-5′-methylphenyl)benzotriazole having a melting point of 128 to 130° C at the yield of 85.0%.

Example 10

97 % sodium hydroxide (8.2 g) was added to methanol (100 ml), and the mixture was stirred at 65° C for 30 minutes. After cooling to 50° C, 2-nitro-4-chloro-2′-hydroxy-3′-t-butyl-5′-methylazobenzene (11.6 g) was added to the resultant mixture over 30 minutes, and thereafter 2,3-dichloro-1,4-naphthoquinone (0.7 g) was added. Glucose (4 g) was then added to the resultant mixture at 40 to 45 ° C over one hour, and the mixture was further stirred for one hour at 40 to 45° C. As this result, almost all of the azobenzene disappeared to produce 2-(2′-hydroxy-3′-t-butyl-5′-methylphenyl)-5-chlorobenzotriazole-N-oxide.

After the reaction, water (50 ml) was added to the reaction liquor, and the resultant reaction liquor was neutralized with 62 % sulfuric acid (10 g) to precipitate a crystal. The precipitated crystal was separated by filtration, and the separated crystal was fully washed with hot water of 60 to 70° C and further with a small amount of methanol. The washed crystal was then dried, thus obtaining 10.6 g of 2-(2′-hydroxy-3′-t-butyl-5′-methylphenyl)-5-chlorobenzotriazole-N-oxide having a melting point of 161 to 163° C at the yield of 96.0 %.

Example 11

The same procedure as in Example 10 was repeated, except that 2-nitro-4-chloro-2′-hydroxy-3′-t-butyl-5′-methylazobenzene (11.6 g) was replaced respectively by (a) 2-nitro-4-chloro-2′-hydroxy-3′, 5′-di-t-butylazobenzene (13.0 g) and (b) 2-nitro-2′-hydroxy-3′, 5′-di-t-amylazobenzene (12.8 g).

The products thus obtained and their properties are as follows:

(a) 2-(2′-hydroxy-3′, 5′-di-t-butylphenyl)-5-chlorobenzotriazole-N-oxide:
Yield: 11.8 g (95.0%), Melting point: 178 to 180° C
(b) 2-(2′-hydroxy-3′, 5′-di-t-amylphenyl)benzotriazole -N-oxide:
Yield: 11.4 g (93.5 %), Melting Point: 110 to 113° C.

Example 12

97 % sodium hydroxide (8.2 g) was added and dissolved in a mixture of methanol (60 ml) and water (20 ml). 2-nitro -4-chloro-2′-hydroxy-3′-t-butyl-5′-methylazobenzene (11.6 g) was then added to the resultant solution at 50 to 60° C over 30 minutes while stirring, and thereafter 2,3-dichloro-1,4-naphthoquinone (0.3 g) and 9-fluorenone (0.4 g) were added to the solution. Glucose (4 g) was then added to the resultant mixture at 40 to 50° C over two hours, and the mixture was stirred for one hour at the same temperature. As a result, almost all of the azobenzene disappeared to produce 2-(2′-hydroxy-3′-t-butyl-5′-methylphenyl)-5-chlorobenzotriazole-N-oxide.

After the reaction, water (50 ml) was added to the reaction liquor, and the resultant reaction liquor was neutralized with 62% sulfuric acid (10 g) to precipitate a crystal. The precipitated crystal was separated by filtration, and the separated crystal was fully washed with hot water of 60 to 70° C and further with a small amount of methanol. The washed crystal was then dried, thus obtaining 10.7 g of 2-(2′-hydroxy-3′-t-butyl-5′-methylphenyl)-5-chlorobenzotriazole-N-oxide having a melting point of 162 to 163° C at the yield of 96.8 %.

Example 13

Water (70 ml), 97% sodium hydroxide (5.2 g), 2-nitro-2′ -hydroxy-5′-methylazobenzene (12.9 g) and toluene (10 ml) were mixed and heated to 60° C. After stirring, hydroquinone (0.6 g) was added and glucose (5.0 g) was added to the mixture over one hour at 40 to 45° C. The mixture was further stirred for two hours,

and the azobenzene disappeared.

The reaction liquor was neutralized with 62 % sulfuric acid 5.8 g. and was cooled to 20 °C to precipitate a crystal. The crystal thus precipitated was separated by filtration and was dried, thus producing 10.8 g of 2-(2′-hydroxy-5′-methylphenyl)benzotriazole-N-oxide having a melting point of 138 to 140 °C at the yield of 90.0 %.

Example 14

97% sodium hydroxide (14.4 g) was added to a mixture of methanol (72 ml) and water (36 ml). 2-nitro-2′-hydroxy-5′ -t-butylazobenzene (15.0 g) was then added to the resultant mixture and the mixture was heated to 45~50 °C. Hydroquinone (0.4 g) and then glucose (5.0 g) were added to the heated mixture over 30 minutes while stirring. The mixture was further stirred for one hour. As a result, the azobenzene disappeared to produce 2-(2′-hydroxy-5′-t-butylphenyl)benzotriazole-N-oxide.

Water (50 ml) was added to the reaction liquor thus obtained, and the liquor was neutralized with 62 % sulfuric acid (23 g) to precipitate a crystal. The precipitated crystal was separated by filtration, and the separated crystal was fully washed with water and further with methanol. The washed crystal was then dried, thus obtaining 12.9 g of 2-(2′-hydroxy-5′-t-butyl phenyl)benzotriazole-N-oxide having a melting point of 73 to 78 °C at the yield of 91.1%.

Example 15

The same procedure as in Example 14 was repeated, except that 2-nitro-2′-hydroxy-5′-t-butylazobenzene (15.0 g) was replaced by 2-nitro-2′-hydroxy-5′-t-octylazobenzene (17.7 g), thus producing 15.2 g of 2-(2′-hydroxy-5′-t-octylphenyl)benzotriazole-N-oxide having a melting point of 106.0 to 111.0 °C at the yield of 90.2 %.

Example 16

A mixture of methanol (60 ml), water (30 ml), 97% sodium hydroxide (12.4 g) and 2-nitro-2′-hydroxy-5′-methyl azobenzene (12.9 g) was heated and stirred at 45~50 °C. 9-fluorenone (1.0 g) and then glucose (5.5 g) were added to the resultant mixture over 30 minutes while stirring. The mixture was further stirred at 75 °C (boiling point) for 7 hours. As a result, the azobenzene disappeared to produce 2-(2′-hydroxy-5′-methylphenyl)benzotriazole-N-oxide.

Water (50 ml) was added to the reaction liquor thus obtained, and the liquor was neutralized with 62 % sulfuric acid (19 g) to precipitate a crystal. The crystal thus obtained was separated by filtration, and the separated crystal was fully washed with water and further with methanol. The crystal thus washed was dried to produce 11.1 g of 2-(2′-hydroxy-5′-methylphenyl)benzotriazole having a melting point of 138 to 140 °C at the yield of 92.1 %.

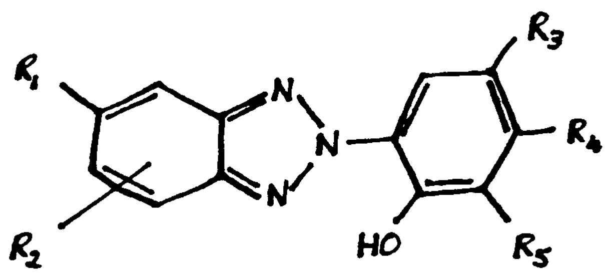

I

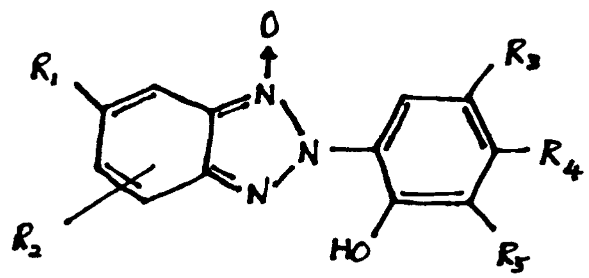

II

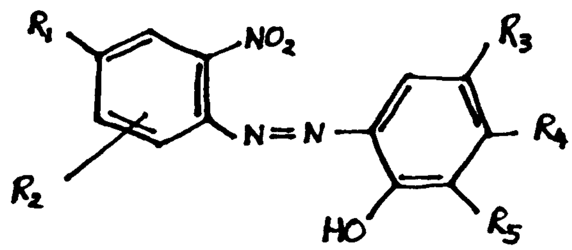

III

## Claims

**1.** A method for preparing a 2-phenylbenzotriazole of formula I

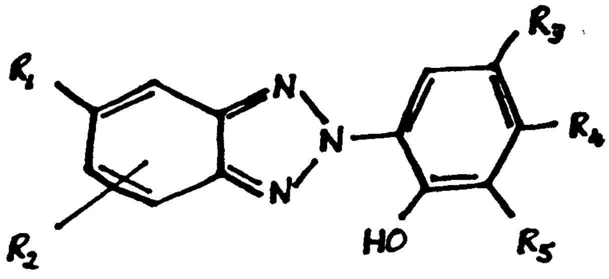

I

wherein $R_1$ is H, Cl, $C_{1-4}$ alkyl, $C_{1-4}$ alkoxy, COOH or $SO_3H$; $R_2$ is H, Cl, $C_{1-4}$ alkyl or $C_{1-4}$ alkoxy;

$R_3$ is H, Cl, $C_{1-12}$ alkyl, $C_{1-4}$ alkoxy, phenyl, ($C_{1-8}$ alkyl)phenyl, phenoxy or phenyl($C_{1-4}$ alkyl); $R_4$ is H, Cl, OH or $C_{1-4}$ alkoxy; and $R_5$ is H, $C_{1-12}$ alkyl or phenyl($C_{1-4}$ alkyl), which comprises reducing a nitroazobenzene of formula III

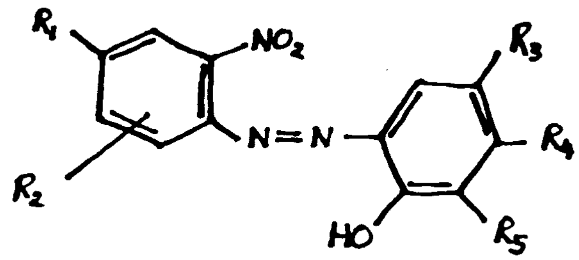

III

wherein $R_1$, $R_2$, $R_3$, $R_4$ and $R_5$, are as defined above, with a saccharide in the presence of a hydrogen transfer catalyst and base.

2. A method according to claim 1, wherein 1 to 2 mol saccharide are used per mol nitroazobenzene (III).

3. A method according to claim 1, wherein, in the first of two steps, 0.5 to 0.8 mol saccharide is used per mol nitroazobenzene (III), to give an intermediate which is a 2-phenylbenzotriazole-N-oxide of formula II

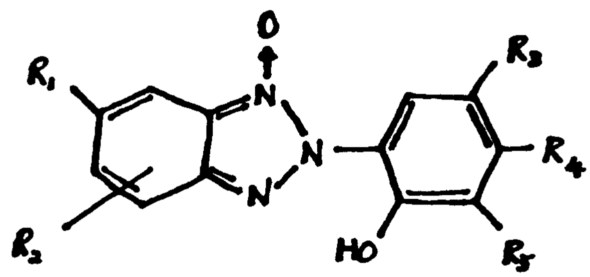

II

wherein $R_1$, $R_2$, $R_3$, $R_4$ and $R_5$ are as defined in claim 1, and, in the second step, 0.5 to 1.5 mol saccharide are used per mol intermediate (II).

4. A method for preparing a 2-phenylbenzotriazole (I) as defined in claim 1, which comprises reducing 2-phenylbenzotriazole-N-oxide (II) as defined in claim 3 with, per mol thereof, 0.5 to 1.5 mol saccharide in the presence of a hydrogen transfer catalyst and base.

5. A method for preparing a 2-phenylbenzotriazole-N-oxide (II) as defined in claim 3, which comprises reducing nitroazobenzene (III) as defined in claim 1 with, per mol thereof, 0.5 to 0.8 mol saccharide in the presence of a hydrogen transfer catalyst and base.

6. A method according to any preceding claim, wherein the saccharide reducing agent is selected from glucose, fructose, sucrose, lactose and maltose.

7. A method according to any preceding claim, wherein the hydrogen transfer catalyst is used in an amount of 0.2 to 30% by weight, based on the weight of the starting material (II or III).

8. A method according to any preceding claim, wherein the hydrogen transfer catalyst is selected from 2,3-dichloro-1,4-naphthoquinone, benzoquinone, anthraquinone, 9-fluorenone, benzanthrone, hydroquinone and 1,4-dihydroxynaphthalene.

9. A method according to any preceding claim, wherein the base is sodium hydroxide or potassium hydroxide in an amount of 1 to 12 mol per mol starting material (II or III).

**Revendications**

1. Procédé pour préparer un 2-phénylbenzotriazole de formule I

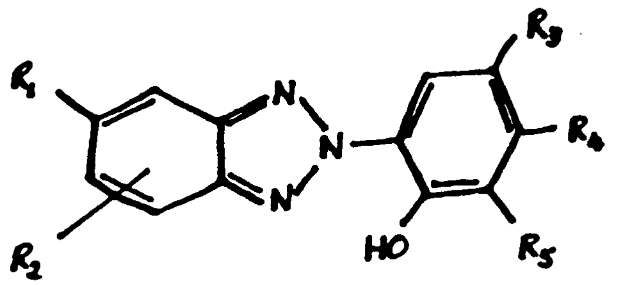

I

dans laquelle $R_1$ est H, Cl, un radical alkyle an $C_{1-4}$, alcoxy en $C_{1-4}$, COOH ou $SO_3H$ ; $R_2$ est H, Cl, un radical alkyle en $C_{1-4}$ ou alcoxy en $C_{1-4}$ ; $R_3$ est H, Cl, un radical alkyle en $C_{1-12}$, alcoxy en $C_{1-4}$, phényle, (alkyl en $C_{1-8}$)phényle, phénoxy ou phényl(alkyle en $C_{1-4}$) ; $R_4$ est H, Cl, OH ou un radical alcoxy en $C_{1-4}$ ; et $R_5$ est H, un radical alkyle en $C_{1-12}$ ou phényl(alkyle en $C_{1-4}$), qui comprend la réduction d'un nitroazobenzène de formule III

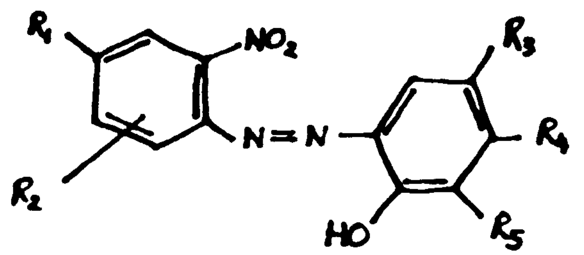

III

dans laquelle $R_1$, $R_2$, $R_3$, $R_4$ et $R_5$ sont tels que définis ci-dessus, avec un saccharide en présence d'un catalyseur de transfert d'hydrogène et d'une base.

2. Procédé selon la revendication 1, dans lequel on utilise 1 à 2 mol de saccharide par mol de nitroazobenzène (III).

3. Procédé selon la revendication 1 dans lequel, dans la première de deux étapes, on utilise 0,5 à 0,8 mol de saccharide par mol de nitrozazobenzène (III) pour obtenir un intermédiaire qui est un 2-phénylbenzotriazole-N-oxyde de formule II

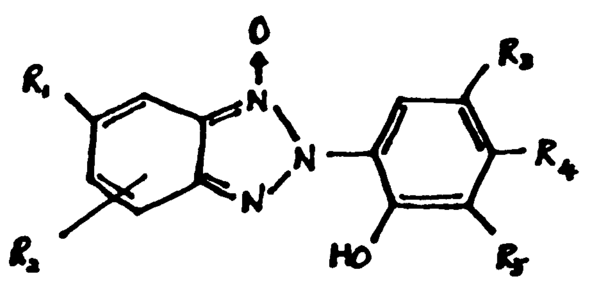

II

dans laquelle $R_1$, $R_2$, $R_3$, $R_4$ et $R_5$ sont tels que définis dans la revendication 1 et, dans une seconde étape, on utilise 0,5 à 1,5 mole de saccharide par mol de l'intermédiaire (II).

4. Procédé pour préparer un 2-phénylbenzotriazole (I) comme défini dans la revendication 1, qui comprend la réduction d'un 2-phénylbenzotriazole-N-oxyde (II) comme défini dans la revendication 3 avec, par mol de celui-ci, 0,5 à 1,5 mol d'un saccharide en présence d'un catalyseur de transfert d'hydrogène et d'une base.

5. Procédé pour préparer un 2-phénylbenzotriazole-N-oxyde (II) comme défini dans la revendication 3, qui

comprend la réduction d'un nitroazobenzène (III) comme défini dans la revendication 1 avec, par mol de celui-ci, 0,5 à 0,8 mol de saccharide en présence d'un catalyseur de transfert d'hydrogène et d'une base.

6. Procédé selon l'une quelconque des revendications précédentes, dans lequel l'agent réducteur saccharidique est choisi parmi le glucose, le fructose, le saccharose, le lactose et le maltose.

7. Procédé selon l'une quelconque des revendications précédentes, dans lequel le catalyseur de transfert d'hydrogène est utilisé en une quantité de 0,2 à 30 % en poids relativement au poids de la matière de départ (II) ou (III).

8. Procédé selon l'une quelconque des revendications précédentes, dans lequel le catalyseur de transfert d'hydrogène est choisi parmi la 2,3-dichloro-1,4-naphtoquinone, la benzoquinone, l'anthraquinone, la 9-fluorénone, la benzanthrone, l'hydroquinone et le 1,4-dihydroxynaphtalène.

9. Procédé selon l'une quelconque des revendications précédentes, dans lequel la base est l'hydroxyde de sodium ou l'hydroxyde de potassium en une quantité de 1 à 12 mol par mol de la matière de départ (II ou III).

## Patentansprüche

1. Verfahren zur Herstellung eines 2-Phenylbenzotriazols der Formel I

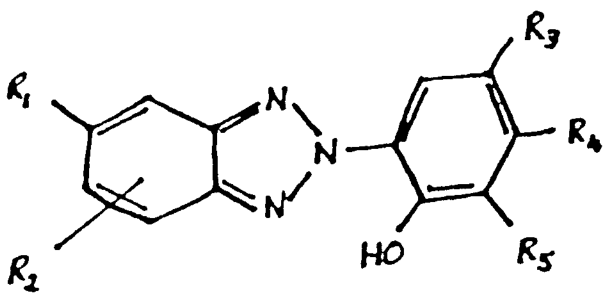

I,

wobei $R_1$ die Bedeutung H, Cl, $C_{1-4}$-Alkyl, $C_{1-4}$-Alkoxy, COOH oder $SO_3H$ hat; $R_2$ die Bedeutung H, Cl, $C_{1-4}$-Alkyl oder $C_{1-4}$-Alkoxy hat; $R_3$ die Bedeutung H, Cl, $C_{1-12}$-Alkyl, $C_{1-4}$-Alkoxy, Phenyl, ($C_{1-8}$-Alkyl)phenyl, Phenoxy oder Phenyl($C_{1-4}$-Alkyl) hat; $R_4$ die Bedeutung H, Cl, OH oder $C_{1-4}$-Alkoxy hat; und $R_5$ die Bedeutung H, $C_{1-12}$-Alkyl oder Phenyl($C_{1-4}$-Alkyl) hat,
wobei man ein Nitroazobenzol der Formel III

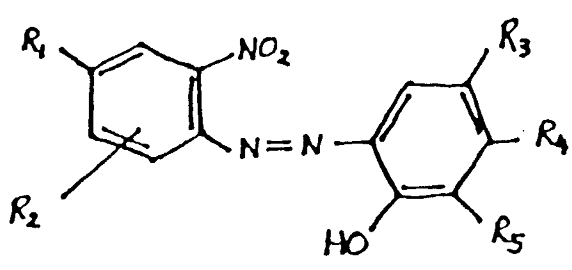

III,

mit einem Saccharid in Gegenwart eines Wasserstoffübertragungskatalysators und Base reduziert, wobei $R_1$, $R_2$, $R_3$, $R_4$ und $R_5$ die vorstehend definierte Bedeutung haben.

2. Verfahren nach Anspruch 1, wobei man 1 bis 2 Mol Saccharid pro Mol Nitroazobenzol (III) einsetzt.

3. Verfahren nach Anspruch 1, wobei man im ersten von zwei Schritten, zur Bildung eines 2-Phenylbenzotriazol-N-oxids der Formel II

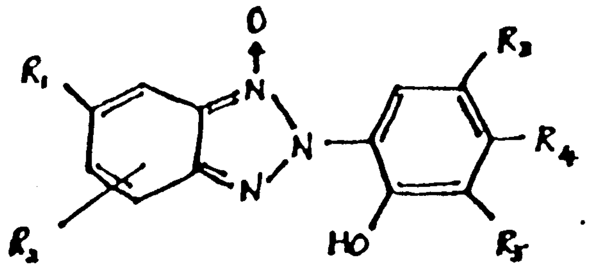

II,

als Zwischenprodukt 0,5 bis 0,8 Mol Saccharid pro Mol Nitroazobenzol (III) einsetzt, wobei $R_1$, $R_2$, $R_3$, $R_4$ und $R_5$ die in Anspruch 1 definierte Bedeutung haben und im zweiten Schritt 0,5 bis 1,5 Mol Saccharid pro Mol Zwischenprodukt (II) einsetzt.

**4.** Verfahren zur Herstellung eines gemäß Anspruch 1 definierten 2-Phenylbenzotriazols (I), wobei man gemäß Anspruch 3 definiertes 2-phenylbenzotriazol-N-oxid (II) mit 0,5 bis 1,5 Mol Saccharid pro Mol davon in Gegenwart eines Wasserstoffübertragungskatalysators und Base reduziert.

**5.** Verfahren zur Herstellung eines gemäß Anspruch 3 definierten 2-Phenylbenzotriazol-N-oxids (II), wobei man gemäß Anspruch 1 definiertes Nitroazobenzol (III) mit 0,5 bis 0,8 Mol Saccharid pro Mol davon in Gegenwart eines Wasserstoffübertragungskatalysators und Base reduziert.

**6.** Verfahren nach einem der vorgehenden Ansprüche, wobei man das Saccharid-Reduktionsmittel auswählt aus Glucose, Fructose, Sucrose, Lactose und Maltose.

**7.** Verfahren nach einem der vorhergehenden Ansprüche, wobei man den Wasserstoffübertragungskatalysator, bezogen auf das Gewicht des Ausgangsstoffes (II oder III), in einer Menge von 0,2 bis 30 Gew.-% einsetzt.

**8.** Verfahren nach einem der vorhergehenden Ansprüche, wobei man den Wasserstoffübertragungskatalysator auswählt aus 2,3-Dichlor-1,4-naphthachinon, Benzochinon, Anthrachinon, 9-Fluorenon, Benzanthron, Hydrochinon und 1,4-Dihydronaphthalin.

**9.** Verfahren nach einem der vorhergehenden Ansprüche, wobei die Base Natriumhydroxid oder Kaliumhydroxid in einer Menge von 1 bis 12 Mol pro Mol Ausgangsstoff (II oder III) ist.